# EUROPÄISCHE PATENTSCHRIFT

(11) **EP 2 884 973 B1**
(45) Veröffentlichungstag und Bekanntmachung des Hinweises auf die Patenterteilung: **10.10.2018**
(21) Anmeldenummer: 13748322.8
(22) Anmeldetag: 14.08.2013
(51) Int. Cl.: A61K 31/191, A61L 15/44, A61K 31/19, A61K 31/192, A61K 31/194, A61P 17/02

(54) **PUFFERSUBSTANZEN ZUR BEHANDLUNG VON WUNDEN**
BUFFER SUBSTANCES FOR THE TREATMENT OF WOUNDS
SUBSTANCES TAMPON POUR LE TRAITEMENT DES PLAIES

(30) Priorität: 16.08.2012 EP 12005908
(43) Veröffentlichungstag der Anmeldung: 24.06.2015
(73) Patentinhaber: Paul Hartmann AG, 89522 Heidenheim (DE)
(72) Erfinder: JUNGINGER, Martin, 89568 Hermaringen (DE)
(74) Vertreter: Ter Meer Steinmeister & Partner
(86) Internationale Anmeldenummer: PCT/EP2013/066972
(87) Internationale Veröffentlichungsnummer: WO 2014/027017

(56) Entgegenhaltungen:
- EP-A2- 0 901 795
- WO-A1-02/28447
- WO-A1-02/47737
- André Friedrich: "Modernes Wundmanagement", 22. Fortbildungstagung Aktuelle Geriatrie Bayreuth, 15. November 2008 (2008-11-15), Seiten 1-56, XP055077624, Internet Gefunden im Internet: URL:http://www.klinikum-bayreuth.de/pdf/pd f_1229376625.pdf [gefunden am 2013-09-04]

## Beschreibung

Die vorliegende Erfindung betrifft Puffersubstanzen zur Behandlung von Wunden, wobei die Behandlung die Unterdrückung der Fibrinbildung und/oder Fibrinvernetzung umfasst. Des Weiteren betrifft die vorliegende Erfindung ein Wundversorgungsprodukt, welches die genannten Puffersubstanzen enthält zur Unterdrückung der Fibrinbildung und/oder Fibrinvernetzung.

Die Heilung von Hautwunden beruht auf der Fähigkeit der Haut, Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Es hat sich gezeigt, dass eine Abheilung der Wunde durch eine feuchte Wundbehandlung besonders gefördert wird. Im Rahmen einer Wundbehandlung können unter anderem Wundauflagen aus verschiedenen Materialien eingesetzt werden.

Es ist bekannt, dass es bei der Wundheilung zu Störungen kommen kann. Im Falle einer gestörten Wundheilung können Nekrosen und pathologische Mikroorganismen auf den physiologischen Metabolismus während des Wundheilungsgeschehens negativ einwirken. Dies kann häufig zu lokaler Hypoxie führen, was dann in einem weiteren Abbau des umliegenden Gewebes resultieren kann. Dieser Abbau des umliegenden Gewebes kann wiederum die Wundheilung zusätzlich behindern, wobei chronische Wunden entstehen können. Als chronische Wunden werden im Rahmen dieser Erfindung Wunden bezeichnet, die nicht in einem erwarteten Zeitraum von 4 bis 6 Wochen verheilen.

Weiterhin können bei der nicht optimalen Behandlung von Wunden Narben zurückbleiben. Im günstigen Fall wird das betroffene Gewebe durch die Narben nur aus kosmetischer Sicht aber nicht in seiner Wirkungsweise beeinträchtigt. Andernfalls ist es aber auch möglich, dass das vernarbte Gewebe funktionelle Eigenschaften, wie seine Elastizität und Empfindlichkeit, einbüßt.

WO 2006/074221 A2 befasst sich mit einem Verfahren zur Reduzierung der Narbenbildung bei einer Wunde. Hierzu wird ein einfach anzuwendendes Mittel beschrieben, welches nach dem Auftragen auf die Wunde einen Anstieg des pH-Wertes in der Wunde herbeiführt. Die alkalisierenden Mittel werden bevorzugt aus einer Gruppe ausgewählt, die Natriumhydrogencarbonat, Natriumhydroxid, Kaliumhydroxid, Tris(hydroxymethyl)-aminomethan (Tromethamin), Acetazolamid (Diamox), Natriumzitrat, Kaliumzitrat, Phosphatsalze (z.B. Natriumphosphat) und Natriumlactat umfasst. Auf diese Weise kann die gewünschte alkalische Umgebung des Wundbereichs herbeigeführt werden, sodass die Aktivität des transformierenden Wachstumsfaktors ("transforming growth factor", β-TGF) gehemmt werden kann, wodurch eine verbesserte Wundheilung und/oder eine Unterdrückung der Narbenbildung erreicht werden soll.

Es hat sich jedoch heraus gestellt, dass sowohl die Wundheilung in dem oben stehenden Verfahren noch verbesserungsfähig ist als auch die Narbenbildung nur unzureichend unterdrückt wird. Auch können die bekannten Wundauflagen zu Hautirritationen führen und sind im Hinblick auf die Therapietreue der Patienten noch optimierbar.

EP 0 901 795 A2 (Johnson & Johnson Medical [US])betrifft ein festes bioabsorbierbares Material, welches ein schwaches, wasserlösliches Säurepuffersystem umfasst, wobei dieses in einer Proteinmatrix in einer Menge von mindestens 0,1 mmol/g, bezogen auf das Gewicht des Materials, gelöst oder dispergiert ist. Das Puffersystem besteht aus einer schwachen, wasserlöslichen und physiologisch unbedenklichen Säure und einer wasserlöslichen, physiologisch verträglichen, konjugierten Base einer schwachen Säure. Das Material wird bevorzugt als oder in einem Wundverband eingesetzt, um Wundflächen in einem sauren pH-Wert zu halten.

WO 02/47737 A1 (Johnson & Johnson Medical Ltd. [GB]; Silcock Derek [GB]; Marsden Donald) offenbart ein geschichtetes Wundverbandmaterial, welches eine der Wunde zugewandte Hydrogelschicht und eine Sperrschicht umfasst, wobei die Sperrschicht ein pH-empfindliches Material enthält. Dieses pH-empfindliche Material ist in Wasser unter sauren Bedingungen bei 25°C im Wesentlichen unlöslich, während es aber in Wasser unter neutralen oder alkalischen Bedingungen bei 25 °C im Wesentlichen löslich ist.

WO 02/28447 A1 (Moelnlycke Health Care AB [SE]; Fabo Thomas [SE]) bezieht sich auf hautfreundliche Klebstoffe. Der Klebstoff enthält eine Substanz, die den pH-Wert bei Kontakt mit der Feuchtigkeit der Haut senkt.

André Friedrich: "Modernes Wundmanagement", 22. Fortbildungstagung, Aktuelle Geriatrie Bayreuth, 15. November 2008, Seiten 1-56, XP0555077624, URL:http://www.klinikum-bayreuth.de/pdf/pdf_1229376625.pdf befasst sich mit dem modernen Wundmanagement, insbesondere mit einer phasengerechten Wundversorgung. Hierbei wird unter anderem darauf hingewiesen, dass eine feuchte Wundbehandlung bzw. ein feuchter Wundverband vorteilhaft seien.

Ziel der vorliegenden Erfindung ist es, die Nachteile aus dem Stand der Technik zu überwinden.

Insbesondere liegt die Aufgabe zugrunde, die Verwendung von Substanzen in der Behandlung von Wunden zur Verfügung zu stellen, welche zu einer verbesserten Wundheilung führen und insbesondere die Narbenbildung vorteilhaft reduzieren oder gar vollständig unterdrücken. Die Substanzen beziehungsweise die diese Substanzen enthaltenden Wundversorgungsprodukte sollen vom Patienten als angenehm empfunden werden und zu einer vorteilhaften Therapietreue führen.

Somit ist es eine Aufgabe der vorliegenden Erfindung, ein verbessertes Wundversorgungsprodukt bereitzustellen, das den pathologischen Zustand einer Wunde derart beeinflusst, dass ein schneller und mit geringer Narbenbildung verbundener Wundheilungsverlauf stattfinden kann. Dieses Wundversorgungsprodukt soll bedarfsgerecht in allen Phasen der Wundheilung angebracht werden können, um den oben genannten Effekt zu gewährleisten.

Es hat sich unerwartet herausgestellt, dass die oben stehenden Aufgaben durch Puffersubstanzen zur Verwendung bei der Behandlung zur Unterdrückung der Fibrinbildung und/oder Fibrinvernetzung sowie durch ein Wundversorgungsprodukt, welches die Puffersubstanzen enthält, gelöst werden können.

Damit sind Gegenstand der Erfindung Puffersubstanzen gemäß Anspruch 1.

Ein weiterer Gegenstand der Erfindung ist ein Wundversorgungsprodukt gemäß Anspruch 7.

Weiterhin wird ein Verfahren zur Behandlung einer Wunde beschrieben, wobei das Verfahren umfasst
(a) Bereitstellen einer Pufferlösung durch Auflösen von Puffersubstanzen in demineralisiertem Wasser und Anpassen des pH-Werts auf einen Wert zwischen 3 und 7, und
(b) phasengerechte Behandlung der Wunde mit der Pufferlösung.

Der Begriff "Puffersubstanzen" bezeichnet im Rahmen der vorliegenden Anmeldung bevorzugt ein Gemisch aus chemischen Substanzen, dessen pH-Wert sich bei der Zugabe einer Base oder einer Säure nicht so stark ändert als in einem ungepufferten System. Die Wirkung dieses gepufferten Systems beruht üblicherweise darauf, dass durch die Zugabe von Oxonium- beziehungsweise Hydroxidionen die entsprechenden schwachen Säuren beziehungsweise Basen entstehen. Die entstandenen schwachen Säuren oder Basen zeigen nur eine geringe Tendenz zur Dissoziation, womit sie ihrerseits nur wenig zur Konzentration von Oxonium- beziehungsweise Hydroxidionen beitragen.

Die Puffersubstanzen können somit als Gemisch aus einer schwachen Säure und einer (korrespondierenden) schwachen Base beziehungsweise als Gemisch aus einer schwachen Base und ihrer (korrespondierenden) schwachen Säure bezeichnet werden. Die Puffersubstanzen können insbesondere aus einer schwachen Säure und dem Salz dieser Säure bestehen.

In einer bevorzugten Ausführungsform der vorliegenden Erfindung bestehen die Puffersubstanzen aus einer organischen Säure, bevorzugt mit 2 bis 12 Kohlenstoffatomen, und dem Salz, bevorzugt dem Alkalisalz, dieser Säure. So können die Gegenionen der korrespondierenden Base zum Beispiel Natriumionen sein.

Die Puffersubstanzen sind bevorzugt nicht-toxische, hautverträgliche und physiologisch unbedenkliche Stoffe. Der pH-Wert der Puffersubstanzen ergibt sich nach Lösung in demineralisiertem Wasser aus dem Protolyse-Gleichgewicht des Pufferpaares aus Säure und Base oder Salz. Der pH-Wert einer Pufferlösung kann in einer guten Näherung gemäß der Henderson-Hasselbalch-Gleichung berechnet werden. Die vorgenannte Gleichung besagt, dass der pH-Wert einer Pufferlösung gleich dem pKs-Wert der Säurekomponente der Pufferlösung plus dem dekadischen Logarithmus des Quotienten der Konzentration der korrespondierenden Base zur Säurekonzentration ist. Aus der Definition der Henderson-Hasselbalch-Gleichung ergibt sich folglich, dass bei gleichen Konzentrationen der Puffersubstanzen der pH-Wert gleich dem pKs-Wert ist. Im Rahmen der vorliegenden Erfindung ist die Verwendung von gleichen Konzentrationen der jeweiligen Puffersubstanzen bevorzugt. In diesem Fall entspricht der pH-Wert der sich bei Lösung der Puffersubstanzen ergebenden Pufferlösung in guter Näherung dem pKs-Wert der Säurekomponente der Puffersubstanzen. Somit kann die Henderson-Hasselbalch-Gleichung genutzt werden, um mittels der pKs-Werte von Puffersubstanzen den pH-Wert der sich ergebenden Pufferlösung abzuschätzen.

Bei einem erfindungsgemäßen Wundversorgungsprodukt wird der pH-Wert, der sich bei Lösung der Puffersubstanzen ergebenden Pufferlösung einstellt, jedoch nicht berechnet, sondern mittels einer pH-Wert Messung bestimmt. Für eine solche Messung werden die Puffersubstanzen mit einer Konzentration von 0,1 M in einem Liter demineralisierten Wasser mit einer Temperatur von 37 °C unter Rühren vollständig gelöst, wodurch sich eine Pufferlösung ergibt. Der pH-Wert der Pufferlösung kann in an sich üblicher Weise mit einem auf Potentiometrie beruhendem handelsüblichen pH-Meter gemessen werden. Bevorzugt wird das Gerät Labor-Daten-pH-Meter CG841 der Firma Schott Geräte GmbH mit einer Glaselektrode Flushtrode der Firma Hamilton Messtechnik GmbH verwendet. Das pH-Meter sollte vor der Messung mittels handelsüblicher Eichlösungen geeicht werden und entsprechend der Bedienungsanleitung des Herstellers verwendet werden. Die Messung des pH-Werts der Pufferlösung erfolgt durch Eintauchen der Messelektrode des pH-Meters in die Pufferlösung, wobei die Pufferlösung gerührt wird. Eine solche Pufferlösung ist dazu geeignet, den pH-Wert einer Flüssigkeit in einem bestimmten pH-Bereich zu stabilisieren, wobei im Rahmen der vorliegenden Erfindung dieser pH-Bereich als Pufferbereich bezeichnet wird. Die vorgenannte Flüssigkeit kann Wundexsudat sein.

Der Pufferbereich einer Pufferlösung ist als ein pH-Bereich mit einer unteren und einer oberen Grenze definiert. Die untere Grenze des Pufferbereichs ist im vorgenannten Fall gleicher Konzentrationen der Puffersubstanzen der pKs-Wert, welcher sich bei einer Lösung der Puffersubstanzen ergebenden Pufferlösung ergibt, minus eine pH-Einheit. Die obere Grenze des Pufferbereichs ist im vorgenannten Fall gleicher Konzentrationen der Puffersubstanzen der pKs-Wert der vorgenannten Pufferlösung plus eine pH-Einheit.

Weiterhin haben Pufferlösungen üblicherweise die Eigenschaft, den pH-Wert bei Zugabe einer Flüssigkeit mit einem anderen pH-Wert als dem pH-Wert der Pufferlösung über längere Zeit im Pufferbereich zu stabilisieren. Ein quantitatives Maß für die vorgenannte pH-Wert-stabilisierende Eigenschaft ist die Pufferkapazität der Pufferlösung.

Die Pufferkapazität ist im Rahmen der vorliegenden Erfindung durch die Menge in mol an NaOH definiert, die in Titrationsexperimenten nötig ist, um den pH-Wert einer Flüssigkeitsmenge von 15 ml demineralisiertem Wasser pro g Produkt, welches Puffersubstanzen enthält, um eine pH-Stufe zu verändern. Die Menge an NaOH wird über den Verbrauch an 0,1M NaOH bestimmt und in mol angegeben.

In einer bevorzugten Ausführungsform der Erfindung ergeben die Puffersubstanzen nach der Lösung in demineralisiertem Wasser bei 37 °C eine Pufferlösung, deren pH-Wert zwischen pH 3 und pH 7, bevorzugt zwischen pH 3,2 und pH 6,5, mehr bevorzugt zwischen pH 3,4 und pH 5,5, insbesondere bevorzugt zwischen pH 3,6 und pH 5,0 liegt. Es hat sich herausgestellt, dass im vorgenannten pH-Wertbereich eine besonders gute Abheilung der Wunde sowie eine Unterdrückung der Fibrinbildung und/oder Fibrinvernetzung erreicht werden kann. Ebenfalls konnte festgestellt werden, dass in diesem Bereich eine besonders vorteilhafte Unterdrückung von Narbengewebe zu beobachten ist.

Beispiele für oben genannte Puffersubstanzen sind Benzoesäure/Benzoat, Milchsäure/Lactat, Glycerinsäure/Glycerat, Gluconsäure/Gluconat, Essigsäure/Acetat, Zitronensäure/Zitrat, Aconitsäure/Aconitat, Glutarsäure/Glutarat, Weinsäure/Tartrat, Äpfelsäure/Malat, Bernsteinsäure/Succinat und Glutaminsäure/Glutamat sowie Mischungen davon. Bei allen genannten Kombinationen liegen die Salze bevorzugt als Alkalisalz, insbesondere als Natriumsalz, vor.

Besonders bevorzugt werden Zitronensäure/Zitrat, Benzoesäure/Benzoat und/oder Milchsäure/Lactat als Puffersubstanzen verwendet.

Im Sinne dieser Erfindung liegt üblicherweise eine Wunde vor, wenn an einer äußeren oder inneren Körperoberfläche der Gewebezusammenhang getrennt wurde.

Es gibt unterschiedliche Arten von Wunden. So wird unter einer primär heilenden Wunde eine Wunde mit nicht klaffenden Wundrändern verstanden, welche sich durch komplikationslose Heilung ohne Infektion auszeichnet. Diese Wunden kommen häufig in gut durchbluteten Körperteilen vor. Die nicht klaffenden und damit eng aneinander liegenden Wundränder können beispielsweise durch einen (chirurgischen) Schnitt verursacht worden sein. Erfolgt keine weitere Behandlung schließt sich die Wunde komplikationslos.

Weitere Wunden sind die sekundär heilenden Wunden. Unter einer sekundär heilenden Wunde wird eine Wunde verstanden, wenn a) ein Verlust des Gewebes vorliegt und/oder b) eine Verkeimung eingetreten ist, welche die primäre Heilung verhindert. Den Verlust an Gewebe kann der Organismus durch neu zu bildendes Gewebe und Überhäutung ausgleichen. Dies führt im Rahmen der sekundären Wundheilung über die Bildung eines Granulationsgewebes bis zum narbigen Ersatz der Gewebslücke.

In einer bevorzugten Ausführungsform der vorliegenden Erfindung ist die sekundär heilende Wunde eine mechanische Wunde, eine thermische Wunde, eine durch chemische Substanzen oder durch Strahlung verursachte Wunde.

Durch äußere Gewalteinwirkung können mechanische Wunden entstehen. Hierzu zählen beispielsweise Schnitt-, Stich-, Platz-, Quetsch-, Schürf-, Kratz-, Biss- und Schusswunden.

Thermische Wunden werden im Wesentlichen durch starke Wärme oder Kälteeinwirkung verursacht. Hierzu zählen beispielsweise Verbrennungen, Verbrühungen, Erfrierungen sowie durch elektrischen Strom verursachte Verletzungen.

Unter chemischen Wunden werden Verätzungen verstanden. Diese können beispielsweise durch saure, alkalische, oxidierende und/oder reduzierende Substanzen verursacht werden.

Durch Strahlung verursachte Wunden werden auch als aktinische Wunden bezeichnet. Diese werden beispielsweise durch ionisierende Strahlung ausgelöst und können ein ähnliches Erscheinungsbild wie Verbrennungswunden aufweisen.

Fibrin ist ein Strukturprotein des Wundheilungsprozesses. Im Wundheilungsprozess kann nach der Fibrinbildung eine Fibrinnetzbildung erfolgen. Der Fibrinbildungsprozess lässt sich vereinfacht auf das Protein Fibrinogen und das Enzym Thrombin zurückführen. Thrombin ist ein in der Leber gebildetes Heterodimer und ist in der Lage Fibrinogen zu Fibrin zu spalten und Gerinnungsfaktoren wie beispielsweise den Faktor XIII zu aktivieren. Nach der Spaltung des Fibrinogens zu Fibrin treten die Fibrinfasern miteinander in Wechselwirkung und es entsteht eine vernetzte Struktur. Diese vernetzte Struktur wird auch als weißer Thrombus bezeichnet, welcher die Wunde verschließt. Durch die weitere Bildung von Fibrin fängt das Fibrinnetz an dreidimensional zu wachsen. Durch den Gerinnungsfaktor XIII kommt es anschließend zur Ausbildung von Quervernetzungen und das Netzwerk gewinnt deutlich an Stabilität. Diese "harte" Kruste wird auch als roter Thrombus bezeichnet.

Nun hat sich unerwartet herausgestellt, dass durch die Unterdrückung der Fibrinbildung und/oder Fibrinvernetzung eine vorteilhafte Wundheilung und eine deutlich verringerte, bevorzugt vollständige, Unterdrückung der Narbenbildung erreicht werden kann.

In einer bevorzugten Ausführungsform der vorliegenden Erfindung wird die Unterdrückung der Fibrinbildung durch die Hemmung des Enzyms Thrombin erreicht. Es wird vermutet, dass der von den Puffersubstanzen vorgegebene pH-Bereich nicht mit dem für das Thrombin charakteristische und eng begrenzte pH-Optimum übereinstimmt. Außerhalb dieses optimalen pH-Bereichs verringert sich gewöhnlich die Reaktionsgeschwindigkeit deutlich oder die vom Enzym gesteuerte Reaktion bleibt aus. Der Grund dafür kann in der durch den pH-Wert bedingten Veränderung der Raumstruktur des Enzyms liegen.

In einer alternativ bevorzugten Ausführungsform der vorliegenden Erfindung wird die Unterdrückung der Fibrinbildung durch die Inaktivierung des Fibrinogens erreicht. Es wird vermutet, dass in dem von den Puffersubstanzen vorgegebenen pH-Bereich das Fibrinogen denaturiert wird und daher nicht in Fibrin gespalten werden kann. In dem vorgegebenen pH-Bereich könnte das Fibrinogen in protonierter Form vorliegen und somit eine veränderte Raumstruktur aufweisen, so dass es enzymatisch nicht vom Thrombin gespalten werden kann.

Gemäß einer bevorzugten Ausführungsform der vorliegenden Erfindung umfasst die Behandlung die Steigerung der zellulären Sauerstoffversorgung der Wunde. Dadurch dass durch die Puffersubstanzen ein für die Wundheilung vorteilhafter pH-Bereich geschaffen und Fibrinbildung und oder Fibrinvernetzung unterdrückt wird, bevorzugt vollständig unterdrückt wird, wird die zelluläre Sauerstoffversorgung der Wunde deutlich gesteigert. Diese gesteigerte zelluläre Sauerstoffversorgung wiederum fördert ihrerseits die das Gewebe aufbauenden (anabolen) Prozesse gegenüber den das Gewebe abbauenden (katabolen) Prozessen und damit eine vorteilhafte Wundheilung mit einer vorteilhaft verringerten Narbenbildung.

Besonders vorteilhaft kann die Narbenbildung durch eine phasengerechte Behandlung der Wunde unterdrückt oder gar vermieden werden.

Die inflammatorische Phase tritt üblicherweise direkt nach dem Trauma auf und dauert in etwa drei Tage. Sie ist gekennzeichnet durch Gefäßkontraktion, Aktivierung der Gerinnungskaskade und komplexe immunologische Abläufe. Es kommt in der Regel zur Ausbildung eines Fibrinnetzes, welches die Wunde verschließt und nach außen schützt. Durch die Freisetzung vasoaktiver Substanzen (z.B. Histamin und Serotonin) kann eine lokale Entzündungsreaktion hervorgerufen werden. Die umliegenden Gefäße können sich erweitern und durch eine gesteigerte Kapillarpermeabilität können Leukozyten zum Entzündungsort wandern. Diese können Mikroorganismen und Gewebsnekrosen beseitigen. Dadurch kann eine Reinigung der Wunde erfolgen.

Die darauffolgende Proliferations- oder Granulationsphase beginnt üblicherweise etwa am zweiten Tag nach der Wundentstehung und kann z.B. bis zu 14 Tage andauern. Es erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Durch das Enzym Plasmin kann das Fibringerüst mittels Fibrinolyse abgebaut werden. Die verschlossenen Gefäße können rekanalisiert werden.

Anders ausgedrückt, die Granulationsphase beginnt üblicherweise mit der Bildung von Granulationsgewebe. Hierfür ist im Allgemeinen eine beginnende Gefäßneubildung (Neoangiogenese) nötig, die sich bevorzugt durch eine sich vergrößernde Rotfärbung des Wundgewebes erkennen lässt. Gleichzeitig wird bevorzugt Matrixmaterial gebildet und das fehlende Gewebe mit gesunden Zellen aufgefüllt. Sobald das Granulationsgewebe seine physiologische Funktionsfähigkeit erreicht hat, kann üblicherweise die nächste Phase der Wundheilung beginnen.

Die Granulationsphase lässt sich folglich vorzugsweise durch die in Augenscheinnahme der Wunde erkennen. Die Wunde in dieser Phase zeigt ein auffällig rot gefärbtes, körniges (granuläres) Gewebe auf der Wundoberfläche, wobei diese Rotfärbung von anderen roten Komponenten wie z.B. Blutkoageln, welche keine Zellen aufweisen, leicht zu unterscheiden ist, insbesondere für medizinisch geschultes Personal. Histologisch ist die Granulationsphase durch die Proliferation neuer kleiner Blutgefäße und die Einwanderung von Fibroblasten, deren Verbund zunehmend dichter wird, zu erkennen.

Der Fachmann (Mediziner) kann somit den Beginn und das Ende der Granulationsphase eindeutig abgrenzen. Zusätzlich sind zur Identifizierung der Granulationsphase weitere Methoden bekannt.

Weiterhin kann die Granulationsphase der Wundheilung durch die Bildung extrazellulärer Matrix, deren wichtigstes Protein Kollagen ist, charakterisiert werden. Kollagen weist normalerweise einen hohen Anteil an posttranslational gebildeten Hydroxyprolin auf. Damit kann die Kollagenbildung zur Bestimmung der Granulationsphase herangezogen werden.

In einer bevorzugten Ausführungsform wird daher die Granulationsphase durch Bestimmung des Kollagengehalt mittels C13-Methode durchgeführt. Mit ¹³C-Kohlenstoff markiertes Prolin wird für die Kollagensynthese verwendet und anschließend zu Hydroxyprolin umgewandelt. Die Bestimmung von markiertem Hydroxyprolin in untersuchtem Gewebe stellt ein gutes Maß für die Kollagensynthese und somit für den Beginn der Granulationsphase dar. Nach der Methode von Babraj JA et al. Am J Physiol Endocrinol Metab: E864-E869, 2005 wird einer Untersuchungsperson eine Dosis von 0,75g [1-¹³C]-Prolin injiziert. Nach 2 Stunden werden eine Probe des zu untersuchenden Wundgewebes sowie eine gleich große Probe intakten Gewebes zum Vergleich entnommen. Es empfiehlt sich, die Vergleichsprobe von einer der Wunde entsprechenden Stelle zu entnehmen, bei einer Oberschenkelwunde z.B. von der der Wunde entsprechenden Stelle am nicht betroffenen zweiten Oberschenkel. Die Gewebeproben werden in flüssigem Stickstoff eingefroren, pulverisiert, mit 0,15 M NaCl-Lösung extrahiert, zentrifugiert, der Überstand wird abgetrennt. Das Pellet wird mit 70%igem Ethanol gewaschen und erneut zentrifugiert. Das isolierte Pellet wird mit über Nacht sauer hydrolysiert. Die freigesetzten Amino- und Iminosäuren werden mittels Dowex 50W-X8 Ionenaustauscher getrennt. Die freigesetzten und getrennten Amino- und Iminosäuren werden als N-Acetyl- n-propylester derivatisiert und zur Messung des Einbaus von markiertem Prolin eingesetzt. Die Bestimmung von ¹³C-markiertem Hydroxyprolin erfolgt bevorzugt durch GC-MS über eine Kalibriergerade mit bekannten Hydroxyprolinkonzentrationen. Der Vergleich der Konzentrationen von ¹³C-markiertem Hydroxyprolin im Wundgewebe und im gesunden Gewebe zeigt, ob eine Kollagensynthese stattgefunden hat.

Eine alternative Methode ist die HPLC-Bestimmung von Glycin, Prolin und Hydroxyprolin nach der in EP 2061898 beschriebenen Methode. Diese Methode bestimmt den Kollagengehalt im Gewebe. Der Vergleich des Gewebekollagengehalts an zwei aufeinander folgenden Messzeitpunkten (z.B. zwei aufeinander folgenden Tagen) gibt Aufschluss darüber, wann neues Kollagen gebildet wird, wann also die Granulationsphase begonnen hat.

Im Rahmen dieser Erfindung wird die Granulationsphase bevorzugt durch in Augenscheinnahme des Fachmanns bestimmt. Sollte dies keine eindeutige Abgrenzung ermöglichen, wird bevorzugt die 13-C Bestimmung angewandt. Sollte auch hier keine eindeutige Abgrenzung möglich sein, dann wird die in EP 2061898 beschriebene Methode verwendet.

In einer bevorzugten Ausführungsform kann die Granulationsphase, insbesondere bei nicht chronischen Wunden, von zweiten Tag bis zum zehnten Tag, bevorzugt vom zweiten bis zum achten Tag, mehr bevorzugt von dritten bis zum siebten Tag, insbesondere vom dritten bis zum sechsten Tag nach Auftreten der Wunde andauern.

In einer alternativen, bevorzugten Ausführungsform kann die Granulationsphase, insbesondere bei chronischen Wunden, von der zweiten Woche oder von der dritten Woche oder von vierten Woche bis zur dem Zeitpunkt dauern, an dem das Granulationsgewebe seine physiologische Funktionsfähigkeit erreicht hat, was mehrere Wochen oder gar Monate, zum Beispiel bis zu 6 Monate, dauern kann.

Mit der Differenzierungs- oder Umbauphase beginnt, etwa zwischen dem sechsten und zehnten Tag, üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es führt zu einer Verkleinerung der Wunde. Die Epithelisierung vom Wundrand her bringt die Wundheilung zum Abschluss.

Die Behandlung der Wunden, bevorzugt der sekundär heilenden Wunden, ist eine phasengerechte Wundbehandlung. Unter einer phasengerechten Wundtherapie wird im Rahmen dieser Anmeldung verstanden, dass die Wundtherapie auf die spezifischen Bedürfnisse der Wunde in den einzelnen Phasen eingeht.

So kann die phasengerechte Behandlung gezielt in einer oder mehreren Phasen der Wundheilung erfolgen. (Im Gegensatz dazu erfolgt bei herkömmlichen Wundbehandlungen ein und dieselbe Behandlung über alle Phasen).

Die Puffersubstanzen gemäß der vorliegenden Erfindung werden ausschließlich in der Granulationsphase verwendet.

Hierfür können beispielsweise die Konzentration der Puffersubstanzen und deren Zusammensetzung bevorzugt so verwendet werden, dass in der jeweiligen Phase eine optimale Wundheilung und die Unterdrückung der Fibrinbildung, welche zu einer vorteilhaften Unterdrückung der Narbenbildung führt, stattfinden kann. Auch kann zum Beispiel die Wunde nach ein bis drei Tagen erneut begutachtet werden und bevorzugt die der aktuellen Wundphase entsprechende Kombination aus den bevorzugten angepassten Puffersubstanzen und deren Konzentration eingesetzt werden.

Weiterhin hat sich gezeigt, dass die pH-Wert stabilisierenden Eigenschaften des Puffers die Ablösung von Nekrosen erleichtern und die Ausbreitung von Keimen in der Wunde verringern können, was den die Wundheilung fördernden Effekt der Unterdrückung der Fibrinbildung in einer sich gegenseitig verstärkenden Weise verbessert.

Das Wundversorgungsprodukt gemäß der vorliegenden Erfindung enthält Puffersubstanzen zur Verwendung in der Behandlung von Wunden zur Unterdrückung der Fibrinbildung und/oder Fibrinvernetzung, wobei die Puffersubstanzen nach Lösung in demineralisiertem Wasser bei 37 °C eine Pufferlösung mit einem pH-Wert zwischen pH 3 und pH 7 bilden.

Im Rahmen dieser Anmeldung sollen alle zu den oben genannten Puffersubstanzen gegebenen Erläuterungen auch für die in dem Wundversorgungsprodukt enthaltenden Puffersubstanzen gelten. So sind beispielsweise die in dem Wundversorgungsprodukt enthaltenen Puffersubstanzen bevorzugt Zitronensäure/Zitrat, Benzoesäure/Benzoat und/oder Milchsäure/Lactat.

In einer bevorzugten Ausführungsform des Wundversorgungsprodukts liegen die Puffersubstanzen in einer Menge von 0,001 bis 10 mmol pro cm², bevorzugt 0,01 bis 5 mmol pro cm², mehr bevorzugt 0,01 bis 1,0 mmol pro cm² des Wundversorgungsprodukts vorliegen. Durch die Menge an Puffersubstanz im Wundversorgungsprodukt kann eine optimale Unterdrückung der Fibrinbildung erreicht werden. Weiterhin kann die Menge im oben genannten Bereich je nach Beurteilung des Zustandes der Wunde bevorzugt so eingestellt werden, dass eine optimale und phasengerechte Behandlung der Wunde durchgeführt werden kann.

Gemäß einer Ausführungsform des erfindungsgemäßen Wundversorgungsprodukts ist es vorteilhaft wenn die Pufferkapazität des Wundversorgungsprodukts bei einer Konzentration der Puffersubstanzen mindestens 0,25 mol bis 5 mol NaOH, insbesondere mindestens 0,55 mol bis 2 mol NaOH beträgt.

Bevorzugt weist das Wundversorgungsprodukt eine der Wunde entsprechend angepasste Form auf. Bevorzugt wird eine im Wesentlichen quadratische Grundform. Besonders bevorzugt ist dabei ein Größenbereich von 1 cm x 1 cm bis zu 30 cm x 30 cm, mehr bevorzugt 5 x 5 cm bis 20 x 20 cm, insbesondere 8 x 8 cm bis 15 x 15 cm. Die Dicke des Wundversorgungsprodukts beträgt bevorzugt von 0,01 cm bis weniger als 2 cm, wobei die Dicke der Wundauflage bevorzugt zwischen 0,1 cm und 1,8 cm liegt.

In einer bevorzugten Ausführungsform umfasst das erfindungsgemäße Wundversorgungsprodukt
(a) eine Wundauflage
(b) optional eine Wundkontaktschicht
(c) optional eine Deckschicht, und
(d) optional eine Klebeschicht.

Unter einer Wundauflage wird im Rahmen dieser Anmeldung ein eine oder mehrere Schichten enthaltendes, bevorzugt steriles, Produkt verstanden, welches auf eine äußere Wunde gelegt werden kann, um das Eindringen von Fremdkörpern in die Wunde zu verhindern und/oder Exsudat aufzunehmen. Vorzugsweise ist die Wundauflage aus einem oder mehreren Materialien aufgebaut, von denen sich vorzugsweise weder Krümel, Fussel noch Fasern ablösen können.

Ein erfindungsgemäßes Wundversorgungsprodukt umfasst bevorzugt eine Wundauflage, welche hydrophile und/oder hydrophobe Materialien bzw. Materiallagen umfasst. Die Verwendung hydrophiler Materiallagen ist für eine schnelle Wundheilung und eine Verringerung der Narbenbildung vorteilhaft, weil solche Wundauflagen Flüssigkeiten aus der Wunde absorbieren können. Hydrophobe Materiallagen können eine rasche Durchleitung von Wundexsudat gewährleisten. Durch eine geeignete Kombination hydrophiler und/oder hydrophober Materialien kann ein feuchtes Wundmilieu aufrechterhalten werden. Dies ist für die Wundheilung förderlich (sogenannte feuchte Wundbehandlung).

In einer besonders bevorzugten Ausführungsform kann die Wundauflage (a) einen Schaumstoff, ein Gittertüll, ein Gewebe, ein Gewirk, ein Gestrick, einen Vliesstoff oder ein Fasermaterial enthalten. In einer alternativen Ausführungsform enthält die Wundauflage (a) beziehungsweise das Wundversorgungsprodukt keinen Schaumstoff.

Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz. So kann die Dichte eines Schaumstoffes, bevorzugt eines Polyurethanschaumstoffes, beispielsweise zwischen 10 und 110 kg/m³, bevorzugt 15 bis 100 kg/m³, mehr bevorzugt 20 bis 95 kg/m³ aufweisen.

In einer weiteren Ausführung der Erfindung ist es weiterhin bevorzugt, Silikonschaumstoffe, z.B. mit einer Dichte von 50 bis 300 kg/m³ einzusetzen.

Bevorzugt wird ein offenzelliger Schaumstoff als Wundauflage (a) verwendet. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt.

Unter Zellwand wird üblicherweise die eine Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf.

Ein geeigneter Schaumstoff kann einen Gradienten der Porengröße über die Dicke des Schaumstoffs aufweisen. Die Porengröße beträgt bevorzugt 1 µm bis 1000 µm, mehr bevorzugt 50 µm bis 800 µm. Die Bestimmung erfolgte mikroskopisch, wobei ein Probenquerschnitt mikroskopiert wurde; die angegebene Porengröße entspricht dem Mittelwert von 5 zufällig ausgewählten und ausgemessenen Poren an der Querschnittsoberfläche pro Probe. Beispielsweise kann das Produkt PermaFoam® der PAUL HARTMANN AG als Wundauflage (a) verwendet werden.

Gittertüll besteht vorzugsweise aus einem hydrophoben Material, beispielsweise Polyester. Der Tüll kann weiterhin mit einer Salbe ausgestattet sein. Besonders geeignete Wundauflagen sind Salbenkompressen der Marke Hydrotüll® und Atrauman® (PAUL HARTMANN AG, Deutschland).

Gewöhnlicherweise werden unter Gewebe Webereierzeugnisse verstanden. Hierzu zählen beispielsweise Tuch, Samt und sonstige textile Flächengebilde aus speziellen Fadenanordnungen, welche im Wesentlichen senkrecht zueinander sind. Die Fäden in Längsrichtung werden Kettfäden genannt und die Fäden in Querrichtung heißen Schussfäden. Um eine ausreichende Festigkeit des Gewebes zu erreichen, müssen die Kett- und Schussfäden dicht miteinander verwoben werden und zeigen deshalb ein geschlossenes Erscheinungsbild.

Gewirke sind auch unter dem Begriff Gewirk oder Wirkwaren bekannt. Gewirke gehören zu den Maschenwaren und sind aus Fadensystemen durch Maschenbildung meist maschinell hergestellte Stoffe.

Unter einem Vliesstoff soll ein Flächen- oder Raumgebilde ausgerichtet angeordneten oder wahllos zueinander befindlichen Fasern verstanden werden, welche mechanisch und/oder thermisch und/oder chemisch verfestigt wurden. Die Vliesstoffe (im Englischem auch "nonwoven") sind wesentlich verschieden von Geweben, Gestricken und Gewirken.

Die Vliesstoffe der vorliegenden Erfindung können Fasern natürlichen oder synthetischen Ursprungs oder Gemische davon enthalten. Zu den Fasern natürlichen Ursprungs zählen z.B. Seide, Cellulose, Baumwolle und Wolle. Die Fasern synthetischen Ursprungs umfassen die synthetischen Polymere (Kunstfasern) wie Viskose, Polyacrylate, Polyamide, Polyimide, Polyamidimide, Polyurethane, Polyester (insbesondere Polyethylenterephthalate und Polybutylenterephthalate), Polyetherester, Polyether, Polyacrylnitrile, Polyalkene (insbesondere Polyethylene und Polypropylene) Polyurethane und Polytetrafluorethylene.

Das Fasermaterial ist bevorzugt ein hydrophiles Fasermaterial. Hierbei können Fasern aus Cellulose, bevorzugt wasserunlösliche Fasern aus Cellulose, insbesondere weitgehend delignifizierte technische Zellstofffasern, insbesondere Holzstofffasern verwendet werden. Insbesondere können Fasern mit einer Faserlänge von < 5 mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten. Es kann auch vorgesehen sein eine Fasermischung aus Cellulose-, regenerierter Cellulose-, Carboxymethylcellulose-, Carboxyethylcellulose-, Hydroxymethylcellulose- oder Hydroxyethylcellulose-Fasern und Fasern aus Polyethylen, Polypropylen oder Polyester zu verwenden.

Die Wundauflage kann zusätzlich ein Partikelgemisch aus Polyacrylatpartikeln enthalten, wobei die Polyacrylatpartikel ein vernetztes und/oder quervernetztes Polyacrylat umfassen.

In einer Fasermaterial enthaltenden Wundauflage ist das Fasermaterial bevorzugt von einer Hüllschicht umgeben.

Eine Fasermaterial enthaltende Wundauflage mit Puffersubstanzen kann mit Puffersubstanzen imprägniertes Fasermaterial und/oder eine mit Puffersubstanzen imprägnierte Umhüllung enthalten.

Bevorzugt wird eine derartige Wundauflage erhalten, indem ein Produkt, umfassend eine Kunststoff-Umhüllung, bevorzugt aus nicht verklebendem Polyamid/Viskose-Vlies, einen Saugkörper, bevorzugt aus Zelluloseflocken, eine Tissuelage zur Sekretverteilung und gegebenenfalls feuchtigkeitsabweisende Vlieslage auf der Rückseite der Wundauflage als Kontaminationsschutz (Zetuvit®) mit den Puffersubstanzen imprägniert wird, zum Beispiel mit den Puffersubstanzen Zitronensäure/Zitrat imprägniert wird.

Alternativ kann entweder der Saugkörper oder die Kunststoff-Umhüllung mit den Puffersubstanzen imprägniert werden bevor das Produkt fertig gestellt wird und als imprägnierte Rohstoffe zu dem fertigen Produkt verarbeitet werden. Dieses Vorangehen empfiehlt sich besonders dann, wenn der Saugkörper superabsorbierende Partikel aus Polyacrylat enthält.

Gemäß einer weiteren Ausführungsform umfasst das erfindungsgemäße Wundversorgungsprodukt eine Wundkontaktschicht (b). Die Wundkontaktschicht ist im Wundversorgungsprodukt bevorzugt zwischen Wundauflage und der Wunde angebracht und ist somit bevorzugt in direktem Kontakt mit der Wunde. Die Wundkontaktschicht gewährleistet bevorzugt bei Wechsel des Wundversorgungsproduktes eine gewebeschonende Ablösung von der Wunde. Die Wundkontaktschicht kann weitere Funktionen in Bezug auf die zu behandelnde Wunde ausüben. Beispielsweise kann die Wundkontaktschicht die Wunde mit Feuchtigkeit versorgen, wundrandpflegende Eigenschaften aufweisen, Hautirritationen vermindern oder antiadhärent wirken.

Die Wundkontaktschicht kann bevorzugt ein Hydrogel, einen Polymerfilm, eine Hydrokolloidmatrix, ein Polymernetz, ein Nonwoven und/oder ein Adhäsiv enthalten. Vorzugsweise umfasst die Wundkontaktschicht ein Hydrogel.

Der Begriff Hydrogel bezeichnet dabei im Rahmen der Erfindung ein feindisperses System aus mindestens einer festen und einer flüssigen Phase. Diese feste Phase bildet dabei ein schwammartiges, dreidimensionales Netzwerk, dessen Poren durch eine Flüssigkeit (Lyogel) beziehungsweise auch ein Gas (Xerogel) ausgefüllt sind. Beide Phasen durchdringen sich dabei bevorzugt vollständig. Durch Wasseraufnahme kann das dreidimensionale Netzwerk durch Quellen sein Volumen vergrößern, ohne dabei den strukturellen Zusammenhalt zu verlieren. Ein Hydrogel kann bevorzugt aus einem synthetischen oder natürlichen Material, bevorzugt aus einem hydrophilen synthetischen Material, aufgebaut sein.

Eine vorteilhafte Rezeptur für ein Hydrogel wird beispielsweise in EP 0 426 422 beschrieben. So ist ein Hydrogel bevorzugt erhältlich durch Umsetzung einer Mischung enthaltend
15 bis 30 Gewichtsprozent eines mehrwertigen Alkohols,
8 bis 14 Gew.-% eines Prepolymers mit Diisocyanatgruppen, bevorzugt Isophorondiisocyanatendgruppen,
0,1 bis 15 Gew.-% eines Diamins, bevorzugt auf Polyethylenoxidbasis,
0 bis 1 Gew.-% eines Salzes,
und als Rest Wasser.
Die Gewichtsangaben beziehen sich hierbei auf das Gesamtgewicht des Hydrogels, bevorzugt jedoch ohne Puffersubstanzen. Die wässrige Phase kann zusätzlich die erfindungsgemäßen Puffersubstanzen enthalten.

Neben Wundauflage (a) und gegebenenfalls Wundkontaktsicht (b) kann das Wundversorgungsprodukt ferner noch eine optionale Deckschicht (c) und/oder eine optionale Klebeschicht (d) umfassen.

Als Deckschicht sind bevorzugt Filme oder Schaumstoffe geeignet, die aus Polyurethan, Polyester, Polyetherurethan, Polyesterurethan, Polyether-Polyamid-Copolymeren, Polyacrylat oder Polymethacrylat gefertigt werden. Insbesondere ist als Trägerschicht ein wasserundurchlässiger und wasserdampfdurchlässiger Polymerfilm geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 60 µm, insbesondere 20 bis 40 µm und ganz besonders bevorzugt von 25 bis 30 µm aufweisen. Die Wasserdampfdurchlässigkeit des Polymerfilms weist vorzugsweise mindestens 300 g/ m²/ 24 Std., insbesondere mindestens 1000 g/ m²/ 24 Std. und ganz besonders bevorzugt mindestens 2000 g/ m²/ 24 Std. bis z.B. 5000 g/ m²/ 24 Std. oder 10.000 g/ m²/ 24 Std. auf (gemessen nach DIN EN 13726, upright).

Die Deckschicht kann mit einer Klebeschicht (d) bedeckt sein. In besonders bevorzugten Ausführungsformen weist die Deckschicht einen feuchtigkeitsdichten, klebenden Randabschnitt auf. Dieser Randabschnitt gewährleistet, dass die Wundauflage an ihrem bestimmungsgemäßen Ort appliziert und fixiert werden kann. Darüber hinaus ist sichergestellt, dass keine Flüssigkeit zwischen der Folie und der die zu behandelnde Fläche umgebende Haut austreten kann. Als besonders bevorzugt sind solche Klebstoffe zu betrachten, die in einem dünnen Auftrag von 20 bis 35 g/ m² zusammen mit dem Film eine Wasserdampfdurchlässigkeit von 400 bis 10.000 g/ m²/ 24 Std. und vorzugsweise von 1000 bis 5000 g/ m²/ 24 Std. (gemessen nach DIN EN 13726, upright) aufweisen.

Wundauflage (a), Deckschicht (c) und gegebenenfalls Klebeschicht (d) können gemeinsam eine sogenannte mehrschichtige Wundauflage bilden.

In dem erfindungsgemäßen Wundversorgungsprodukt sind die erfindungsgemäßen Puffersubstanzen bevorzugt in der Wundauflage, in der Wundkontaktschicht oder in der Wundauflage und der Wundkontaktschicht enthalten. Die Puffersubstanzen können in fester oder flüssiger Form enthalten sein. Bevorzugt sind die Puffersubstanzen in fester Form in dem Wunderversorgungsprodukt enthalten.

In einer bevorzugten Ausführungsform können die im Wundversorgungsprodukt enthaltenen Puffersubstanzen von den Flüssigkeiten der Wunde gelöst werden. Die so entstandene Lösung kann in die Wunde diffundieren und dort den pH-Wert im sauren Bereich stabilisieren und die Fibrinbildung unterdrücken. Da außerdem die in dem Wundversorgungsprodukt absorbierte Flüssigkeit im sauren pH-Bereich gepuffert wird, wird auch bei dieser Variante zusätzlich die Vermehrung von Keimen, die ein alkalisches Milieu bevorzugen, vermindert. Somit wird eine Rückkontamination der Wunde mit Keimen verhindert und die Intervalle für Verbandwechsel können bei Bedarf verlängert werden.

Besonders bevorzugt ist eine gleichmäßige Verteilung der Puffersubstanzen in Wundauflage und/oder Wundkontaktschicht. So kann beispielsweise eine Wundauflage, welche Puffersubstanzen enthält mit einer Wundkontaktschicht, bevorzugt einem Hydrogel, welches Puffersubstanzen enthält, kombiniert werden. Diese Kombination gewährleistet eine besonders hohe Pufferkapazität und eine konstante Anfeuchtung der Wunde, wodurch eine gute Wundheilung, eine besonders wirksame Unterdrückung der Fibrinbildung und damit eine vorteilhafte Vermeidung von Narbenbildung erreicht werden kann.

Gemäß einer besonders bevorzugten Ausführungsform der Erfindung umfasst das Wundversorgungsprodukt eine Pufferlösung, welche die Puffersubstanzen Zitronensäure und Zitrat, insbesondere Na-Zitrat umfasst.

Gemäß einer anderen Ausführungsform der Erfindung umfasst das Wundversorgungsprodukt die Puffersubstanzen Milchsäure und Lactat. Dieses Puffersystem ist vorteilhaft, weil es mit einem pKs von 3,9 einen besonders niedrigen pH-Wert stabilisieren kann.

Gemäß einer weiteren Ausführungsform der Erfindung umfasst das Wundversorgungsprodukt Benzoesäure und Benzoat als Puffersubstanzen. Diese Pufferlösung ist vorteilhaft, weil sie mit einem pKs von 4,2 einen physiologisch für die normale Wundheilung besonders vorteilhaften pH-Wert stabilisieren und die Fibrinbildung unterdrücken kann.

Gemäß einer bevorzugten Ausführungsform der Erfindung erreicht die Pufferkapazität des Wundversorgungsprodukts nach spätestens drei Stunden, bevorzugt nach 1 bis 2,5 Stunden, 90% der nach 24 Stunden messbaren maximalen Pufferkapazität. Die vorgenannte schnelle Lösung der Puffersubstanzen ist besonders vorteilhaft, weil das Wundversorgungsprodukt bei einer Anwendung auf einer Wunde schnell eine hohe Pufferkapazität bereitstellen kann. Die schnelle Bereitstellung einer hohen Pufferkapazität ermöglicht es, den pH-Wert in der Wunde von Beginn der Behandlung an zuverlässig im sauren pH-Bereich zu stabilisieren.

Es hat sich gezeigt, dass die Wundheilung, die Unterdrückung der Fibrinbildung und die vorteilhafte Verringerung der Narbenbildung bei Anwendung des erfindungsgemäßen Wundversorgungsproduktes in besonders vorteilhafter Weise beeinflusst werden. Hierbei scheinen die an sich bekannten, die Wundheilung fördernden Eigenschaften von Wundauflagen und gegebenenfalls der Einsatz von Hydrogel mit den pH-Wertstabilisierenden Eigenschaften der Puffersubstanzen in einer sich gegenseitig verstärkenden Weise zusammenzuwirken.

Generell ist eine lange Verweildauer einer mit dem Wundgrund direkt in Kontakt tretenden Komponente des Wundversorgungsproduktes auf der Wunde erwünscht, da jeder Wundversorgungsproduktwechsel den Wundheilungsprozesses negativ beeinträchtigen kann. Weiterhin ist der Wundversorgungsproduktwechsel unangenehm für den Patienten und erhöht den Aufwand für das behandelnde medizinische Personal. Wundversorgungsprodukte mit Wundauflagen aus z.B. Vliesstoffen oder Schaumstoffen sind für lange Verweildauern auf der Wunde bevorzugt geeignet, denn sie verfügen über ein hohes Absorptionsvermögen für Flüssigkeiten in der Wunde. Weiterhin konnte beobachtet werden, dass der Puffereffekt über die Verweildauer des Wundversorgungsproduktes auf der Wunde weitgehend erhalten bleibt.

In einer bevorzugten Ausführungsform des Wundversorgungsprodukts wird ein besonders wirksames Micro-Debridement ermöglicht. Unter Micro-Debridement wird das schonende Entfernen von Wundexsudat und zerstörtem Gewebe aus der Wunde beim Verbandwechsel bezeichnet. Das "Micro-Debridement" unterstützt das zügige Abheilen der Wunde, und mit der gleichzeitigen Unterdrückung der Fibrinbildung wird eine bevorzugt verringerte Narbenbildung ermöglicht. Es hat sich gezeigt, dass die pH-Wert-stabilisierenden Eigenschaften des Wundversorgungsprodukts die Fibrinbildung unterdrücken und zusätzlich die Ablösung von Nekrosen erleichtern und die Ausbreitung von Keimen in der Wunde verringern können, was den die Wundheilung fördernden und die Narbenbildung reduzierenden Effekt einer Wundauflage in einer sich gegenseitig verstärkenden Weise verbessert.

Weiterhin beschreibt die Anmeldung ein Verfahren zur Herstellung eines Wundversorgungsprodukts. Das Verfahren zur Herstellung eines Wundversorgungsprodukts umfasst das Zurverfügungstellen einer Wundauflage, das Zurverfügungstellen von Puffersubstanzen, wobei der pH-Wert der entsprechenden Pufferlösung bei 37 °C zwischen pH 3 und pH 7 liegt. Weiterhin umfasst das Verfahren gegebenenfalls das Zurverfügungstellen optionaler weiterer Schichten, wie beispielsweise einer Wundkontaktschicht.

Anschließend wird die Wundauflage mit der Pufferlösung imprägniert und anschließend entweder vollständig oder teilweise getrocknet. Zur Imprägnierung wird die Wundauflage beispielsweise für mindestens 60 Sekunden in eine Puffersubstanzen enthaltende Pufferlösung, die Puffersubstanzen mit einer Gesamtkonzentration von 0,01 bis 0,6 M enthalten kann, getaucht. Im Rahmen der vorliegenden Erfindung bezeichnet der Begriff Konzentration dabei jeweils die Gesamtkonzentration aller eingesetzten Puffersubstanzen. Danach wird bevorzugt die Wundauflage abgetropft und anschließend für drei Tage bei Normklima (23 °C, 50% relative Luftfeuchtigkeit, siehe DIN EN ISO 139) vollständig getrocknet. Für eine teilweise Trocknung der Wundauflage kann eine kürzere Trocknungszeit gewählt werden. Nach dem Imprägnieren der Wundauflage mit der Pufferlösung umfasst das Verfahren weiterhin das Zusammenfügen der imprägnierten Wundauflage und der weiteren Schichten zu einer mehrschichtigen Wundauflage. Dieses Verfahren ist bevorzugt dafür geeignet, einem Wundauflagenmaterial nach dessen Herstellung pH-Wert-verändernde Eigenschaften zu verleihen.

Gemäß einer weiteren vorteilhaften Ausführungsform betrifft die Erfindung ein Verfahren zur Herstellung eines Wundversorgungsprodukts, bei dem die Puffersubstanzen in der Wundkontaktschicht, bevorzugt in einem Hydrogel, enthalten sind. In diesem Verfahren werden Puffersubstanzen in demineralisiertem Wasser gelöst und mit dem Hydrogel vermischt, so dass sich eine Konzentration von 0,01 bis 0,6 M Puffersubstanzen für die Mischung ergibt. Weiterhin umfasst das Verfahren das Zusammenfügen der Wundauflage und des Hydrogels, wobei das Hydrogel die Wundkontaktschicht des Wundversorgungsprodukts bildet.

Nachstehend werden Ausführungsformen erfindungsgemäßer Wundversorgungsprodukte anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen. Es zeigen:
**Figur 1****:** Querschnitt durch ein mehrschichtiges Wundversorgungsprodukt
**Figur 2****:** Querschnitt durch eine weitere Ausführungsform eines mehrschichtigen Wundversorgungsproduktes
**Figur 3****:** Draufsicht auf die im Gebrauch der Wunde zugewandte Seite des mehrschichtigen Wundversorgungsprodukts aus Figur 1

| **Bezugszeichen** | **Bedeutung** |
|---|---|
| 10 | Wundversorgungsprodukt |
| 11 | Wundauflage |
| 12 | Deckschicht |
| 13 | Klebstoffschicht |
| 15 | Wundkontaktschicht, z.B. Hydrogel |
| 16 | Kleberand |
| 20 | Wundversorgungsprodukt, z.B. mit Hydrogel |

### Figur 1

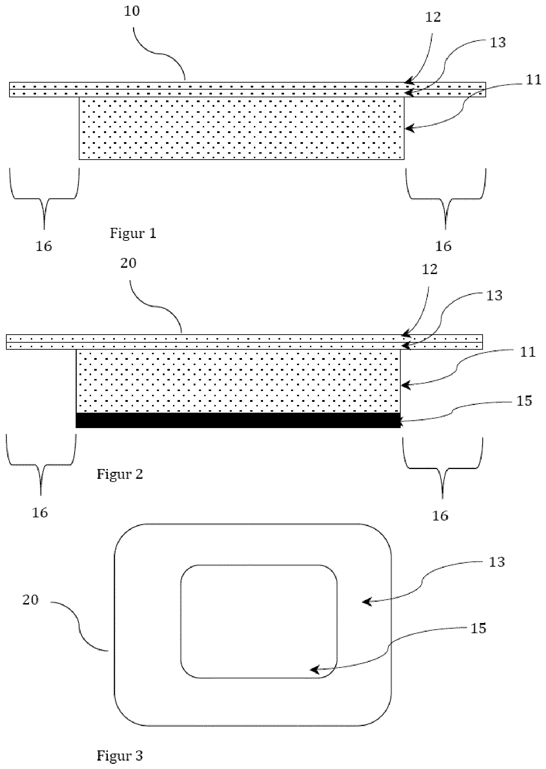

In der in Figur 1 schematisch dargestellten Ausführungsform der Erfindung umfasst das Wundversorgungsprodukt 10 eine mehrschichtige Wundauflage. Diese weist als Trägermaterial eine elastische, Wasserdampf-durchlässige Deckschicht 12 (z.B. aus Polyurethan) auf, die auf einer Seite mit einer Klebeschicht 13 (enthaltend z.B. einen Acrylatkleber) versehen ist. Die Deckschicht 12 ragt über eine Wundauflage 11 hinaus und bildet so einen Kleberand 16, mit dem das Produkt an der Haut des Patienten fixiert werden kann. Die Deckschicht 12 weist vorzugsweise Abmessungen von 15 cm x 15 cm auf, wobei mittig auf der mit Kleber 13 versehenen Seite der Deckschicht 12 die Wundauflage 11 mit einer Größe von 10 x 10 cm vorgesehen ist.

Die Wundauflage 11 wurde durch Imprägnierung, z.B. mit den Puffersubstanzen Benzoesäure und Benzoat (Konzentration 0,04 M), versehen, um eine Stabilisierung des pH-Werts im pH-Bereich pH 4 bis pH 4,5 und eine Pufferkapazität von etwa 0,3 - 0,4 mol NaOH zu erreichen. Nach der Imprägnierung wurde bevorzugt eine vollständige Trocknung der Wundauflage 11 vorgenommen, so dass das Wundversorgungsprodukt 10 vor der Benutzung trocken vorliegt.

Alternativ bevorzugt wäre es außerdem, feuchte Wundauflagen, welche beispielsweise Vliesstoffe oder Schaumstoffe als Wundauflagen enthalten, herzustellen, welche insbesondere zur Behandlung von trockenen Wunden geeignet sind. In einer bevorzugten Ausführungsform des Wundversorgungsprodukts 10 wird die Wundauflage 11 nach dem Imprägnieren nicht vollständig getrocknet, sondern weist nach dem Trocknungsvorgang noch Pufferlösung auf. In diesem Fall kann ein kürzerer Zeitraum für die Trocknung des Schaumstoffs nach dem Imprägnieren vorgesehen werden, wodurch die Wundauflage eine Restfeuchte aufweist. Die Restfeuchte der Wundauflage 11 beträgt bevorzugt zwischen 4 g und 12 g Flüssigkeit pro g Wundauflage.

Wundauflagen (a) mit einer Restfeuchte können im Vergleich mit trockenen Wundauflagen eine geringere Absorptionskapazität aufweisen. Trockene Polyurethanschaumstoffe 11 können schnell große Mengen Wundexsudat aufnehmen, insbesondere wenn keine freisetzbaren Puffersubstanzen enthalten sind. Die schnelle Absorption von Wundexsudat kann zu einer trockenen Wundoberfläche führen. Trockene Wundoberflächen behindern gegebenenfalls einen schnellen Wundheilungsverlauf und können die Narbenbildung begünstigen. Somit sind Wundversorgungsprodukte 10 mit feuchten Wundauflagen (a) hervorragend geeignet, in der Epithelisierungs- oder Granulationsphase der Wundheilung eingesetzt zu werden. Damit kann die erfindungsgemäße Wundauflage 10 in natürlicher Weise die Granulation und/oder die Epithelisierung der Wunde in besonderem Maße fördern.

### Figur 2

Gemäß der in Figur 2 gezeigten Ausführungsform der Erfindung umfasst das Wundversorgungsprodukt 20 ein Hydrogel 15, welches zwischen Wunde und Wundauflage 11 angeordnet ist. Das vorgenannte Hydrogel 15 weist bevorzugt eine Gesamtkonzentration von 0,01 bis 0,1 M Puffersubstanzen, z.B. 0,05 M Zitronensäure und Zitrat auf. Das Hydrogel 15 ist somit geeignet, den pH-Wert einer Wunde im Bereich zwischen pH 4 bis pH 6 zu stabilisieren. Das Wundversorgungsprodukt 20 weist bevorzugt eine Pufferkapazität von 0,1 bis 0,2 mol NaOH auf. Vorteilhaft ist es, wenn das Hydrogel 15 10 bis 20 Gew.-% Glycerin, 0,5 bis 3 Gew.-% Hydroxycellulose und 0,2 bis 2 Gew.-% Natriumchlorid umfasst. Die Deckschicht 12 (bevorzugt eine Polyurethandeckschicht) weist z.B. Abmessungen von 11 cm x 11 cm auf, wobei sich mittig auf der mit Kleber 13 versehenen Seite der Deckschicht 12 eine Wundauflagenschicht 11 mit einer Größe von z.B. 6 x 6 cm befindet.

Gemäß einer Ausführungsform des Wundversorgungsprodukts 20 wird ein Hydrogel 15 verwendet, welches 20 bis 60 Gew.-% Propylenglykol, 3 bis 10 Gew.-% eines Diamins, bevorzugt auf Polyethylen- oder Polyethylenoxidbasis, 0,5 bis 1,5 Gew.-% NaCl und 5 bis 15 Gew.-% Isocyanat umfasst.

Gemäß einer weiteren Ausführungsform des Wundversorgungsprodukts 20 umfasst das Hydrogel 15 die Puffersubstanzen Milchsäure und Lactat in einer Gesamtkonzentration von 0,05 M und kann im pH-Bereich pH 3,5 bis pH 4,2 mit einer Pufferkapazität von in etwa 0,1 bis 0,15 mol NaOH den pH-Wert puffern.

In einer Weiterbildung des in Figur 2 gezeigten Wundversorgungsprodukts 20 können die Wundauflage 11 und das Hydrogel 15 Puffersubstanzen enthalten. Durch die Kombination aus einem Polyurethanschaumstoff 11 und einem Hydrogel 15, wobei sowohl der Polyurethanschaumstoff 11 als auch das Hydrogel 15 Puffersubstanzen für die Stabilisierung des pH-Werts einer Wunde im sauren pH-Bereich aufweisen, kann eine besonders hohe Pufferkapazität erreicht werden.

### Figur 3

Die Wundseite des Wundversorgungsprodukts 20 aus Figur 2 ist in der Draufsicht abgebildet.

Im Folgenden werden Versuche zu den pH-Wert stabilisierenden Eigenschaften erfindungsgemäßer Wundversorgungsprodukte erläutert.

Bei einem erfindungsgemäßen Wundversorgungsprodukt, welches Puffersubstanzen und eine Wundauflage mit einer Schaumstoffschicht umfasst, kann die Abgabe von Puffersubstanzen in die Wunde durch ein einfaches Testsystem mittels in-vitro Titrationsexperimenten gemessen werden. Die Fähigkeit des erfindungsgemäßen Wundversorgungsprodukts, einen pH-Wert in einem sauren pH-Bereich zu stabilisieren, wird mittels der Pufferkapazität beschrieben. Je höher die Pufferkapazität ist, desto mehr Wundexsudat kann durch das erfindungsgemäße Wundversorgungsprodukt im sauren pH-Bereich stabilisiert werden, wobei die Fibrinbildung und/oder Fibrinvernetzung vorteilhaft verhindert wird.

Zur Messung der Pufferkapazität des in dem Wundversorgungsprodukt enthaltenen Schaumstoffs wurde 1 g der Schaumstoffschicht des erfindungsgemäßen Wundversorgungsprodukts in ein Wasserbad mit 15 ml demineralisiertem Wasser gelegt. Das Wasserbad mit dem Wundversorgungsprodukt wurde 24 Stunden lang bei Raumtemperatur (25 °C) auf einem Schüttler mit 100 bpm (Schüttelbewegungen pro Minute) geschüttelt. Die Puffersubstanzen konnten sich während der 24 Stunden aus der Schaumstoffschicht in das demineralisierte Wasser lösen und eine Flüssigkeit mit pH-Wert-stabilisierenden Eigenschaften bilden. Anschließend wurde die Flüssigkeit, welche Puffersubstanzen enthielt, zusammen mit dem Produkt automatisiert titriert. Durch den Verbrauch an NaOH konnte die Pufferkapazität bestimmt werden. Es wurden jeweils Dreifachbestimmungen durchgeführt. Es konnte beobachtet werden, dass der pH-Wert-stabilisierende Effekt über die Versuchsdauer von 24 Stunden weitgehend erhalten bleibt.

Die Pufferkapazität des in dem Wundversorgungsprodukt enthaltenen Hydrogelanteils, sofern vorhanden, wurde auf analoge Weise ermittelt, d.h. es wurde 1 g der Gellage in ein Wasserbad mit 15 ml demineralisiertem Wasser gelegt. Die Freisetzung der Puffersubstanzen aus der Gellage und die Bestimmung der Pufferkapazität erfolgte wie vorstehend für die Schaumstoffschicht beschrieben.

Als vorteilhaft für eine gewebeschonende Stabilisierung des pH-Werts im sauren pH-Bereich haben sich in Vorexperimenten Konzentrationen der Puffersubstanzen von 0,01 M bis 0,6 M erwiesen. Es zeigte sich, dass ein Polyurethanschaumstoff (PermaFoam® von PAUL HARTMANN AG) eine besonders hohe Pufferkapazität von 0,72 +/- 0,05 mol NaOH aufwies (siehe Tabelle 1) wenn für die Imprägnierung die Puffersubstanzen Zitronensäure und Zitrat (Konzentration 0,05 M) verwendet wurden. Die Puffersubstanzen Benzoesäure und Benzoat (Konzentration 0,04 M) wiesen eine geringere Pufferkapazität von 0,33 +/- 0,02 mol NaOH auf. Die Puffersubstanzen Milchsäure / Lactat (Konzentration 0,05 M) wiesen gleichfalls eine geringere Pufferkapazität von 0,30 mol +/- 0,01 mol NaOH auf.

**Tabelle 1: Pufferkapazität von mit Puffersubstanzen imprägnierten Schaumstoffschichten und mit Puffersubstanzen versetzten Gelen in Abhängigkeit von den verwendeten Puffersubstanzen und der Konzentration der Puffersubstanzen.**

| **Wund auflage** | **Puffersubstanzen** | **Konzentration der Puffersubstanzen [M]** | **Pufferkapazität [mol NaOH]** | **+/σ [mol NaOH]** |
|---|---|---|---|---|
| Polyurethanschaumstoff | Zitronensäure/ Zitrat | 0,05 | 0,72 | 0,05 |
| Polyurethanschaumstoff | Zitronensäure/ Zitrat | 0,10 | 1,51 | 0,02 |
| Polyurethanschaumstoff | Benzoesäure/ Benzoat | 0,02 | 0,16 | 0,01 |
| Polyurethanschaumstoff | Benzoesäure/ Benzoat | 0,04 | 0,33 | 0,02 |
| Polyurethanschaumstoff | Milchsäure/ Lactat | 0,05 | 0,30 | 0,01 |
| Polyurethanschaumstoff | Milchsäure/ Lactat | 0,10 | 0,62 | 0,05 |
| Hydrogel | Zitronensäure/ Zitrat | 0,05 | 0,13 | 0,01 |
| Hydrogel | Milchsäure/ Lactat | 0,10 | 0,11 | 0,01 |

| | | | | |
|---|---|---|---|---|
| σ: empirische Standardabweichung. | | | | |

Anstelle des Schaumstoffs kann in derselben Weise ein Produkt hergestellt werden, das als Saugkörper ein Nonwoven (Vlies) auf Cellulosebasis enthält (z.B. Hydrofilm® plus). Hierbei handelt es sich bevorzugt um einen Nonwoven Typ LM0027 der Firma Freudenberg. Dieses Produkt weist nach Imprägnierung eine Pufferkapazität von 0,6 mmol/g, beziehungsweise von 0,011 mmol/cm², auf.

In weiteren Messungen wurde bestimmt, wie schnell sich die Puffersubstanzen aus den Schaumstoffschichten und Hydrogelen in demineralisiertem Wasser lösen können. Hierzu wurde eine 1 g Probe der Schaumstoffschicht bzw. des Hydrogels des erfindungsgemäßen Wundversorgungsprodukts in 15 ml demineralisiertes Wasser gegeben und das Wasserbad mit dem Wundversorgungsprodukt bei Raumtemperatur (25 °C) auf einem Schüttler mit 100 bpm (Schüttelbewegungen pro Minute) geschüttelt. Für jede Messreihe wurden vier Proben in je einem Wasserbad geschüttelt. Die Messreihen wurden zuerst an Schaumstoffschichten durchgeführt.

Bei der ersten Probe wurde die Pufferkapazität der Flüssigkeit, welche Puffersubstanzen enthielt, im ersten Wasserbad mittels Titration mit NaOH nach einer Stunde gemessen. Es zeigte sich, dass sich bei der ersten Probe, also bereits nach einer Stunde, mindestens 90% der nach 24 Stunden messbaren maximalen Pufferkapazität entfaltet hatte. Nach einer Stunde waren also mehr als 90% der aus dem Schaumstoff in die Flüssigkeit lösbaren Puffersubstanzen in der Flüssigkeit gelöst.

Bei der zweiten Probe im zweiten Wasserbad wurde die Titration nach 3 Stunden durchgeführt. Die Pufferkapazität betrug über 90% der nach 24 Stunden messbaren maximalen Pufferkapazität.

Bei der dritten Probe im dritten Wasserbad wurde die Titration nach 6 Stunden durchgeführt. Die Pufferkapazität betrug gleichfalls über 90% der nach 24 Stunden messbaren maximalen Pufferkapazität.

Bei der vierten Probe im vierten Wasserbad wurde die Titration nach 24 Stunden durchgeführt. Die bei dieser Messung bestimmte Pufferkapazität wurde als 100% der nach 24 Stunden messbaren maximalen Pufferkapazität festgesetzt.

Im Rahmen der Messreihe an Schaumschichten zeigte sich, dass die Pufferkapazität bei den Messungen nach 1, 3 und 6 Stunden auf einem Plateau mit über 90% der nach 24 Stunden messbaren maximalen Pufferkapazität konstant blieb.

Bei den mit Puffersubstanzen versetzten Gelen wurden in analoger Weise Titrationsexperimente durchgeführt. Nach drei Stunden wurde eine vollständige Lösung der Puffersubstanzen aus dem Gel in der Flüssigkeit festgestellt, wobei die Pufferkapazität auch bei den dritten und vierten Proben, also nach 6 Stunden und 24 Stunden, konstant blieb.

## Patentansprüche

1. Puffersubstanzen zur Verwendung in der Behandlung von Wunden, wobei die Behandlung als phasengerechte Wundbehandlung ausschließlich in der Granulationsphase erfolgt und die Unterdrückung der Fibrinbildung und/oder Fibrinvernetzung umfasst, und wobei die Puffersubstanzen nach Lösung in demineralisiertem Wasser bei 37°C eine Pufferlösung mit einem pH-Wert zwischen pH 3 und pH 7 bilden.

2. Puffersubstanzen zur Verwendung nach Anspruch 1, wobei die Behandlung die Steigerung der zellulären Sauerstoffversorgung der Wunde umfasst.

3. Puffersubstanzen zur Verwendung nach einem der vorstehenden Ansprüche, wobei die Behandlung die Hemmung von Thrombin umfasst.

4. Puffersubstanzen zur Verwendung nach einem der vorstehenden Ansprüche, wobei die Wunde eine sekundär heilende Wunde, bevorzugt eine mechanische Wunde, eine thermische Wunde, eine durch chemische Substanzen oder durch Strahlung verursachte Wunde, ist.

5. Puffersubstanzen zur Verwendung nach einem der vorstehenden Ansprüche, wobei die Puffersubstanzen aus einer organischen Säure mit 2 bis 12 Kohlenstoffatomen und dem Salz, bevorzugt dem Alkalisalz dieser Säure, bestehen.

6. Puffersubstanzen zur Verwendung nach einem der vorstehenden Ansprüche, wobei die Puffersubstanzen ausgewählt sind aus Benzoesäure/Benzoat, Milchsäure/Lactat, Glycerinsäure/Glycerat, Gluconsäure/Gluconat, Essigsäure/Acetat, Zitronensäure/Zitrat, Aconitsäure/Aconitat, Glutarsäure/Glutarat, Weinsäure/Tartrat, Äpfelsäure/Malat, Bernsteinsäure/Succinat und Glutaminsäure/Glutamat, sowie Mischungen davon.

7. Wundversorgungsprodukt enthaltend Puffersubstanzen zur Verwendung in der Behandlung von sekundär heilenden Wunden ausschließlich während der Granulationsphase zur Unterdrückung der Fibrinbildung und/oder Fibrinvernetzung, wobei die Puffersubstanzen nach Lösung in demineralisiertem Wasser bei 37°C eine Pufferlösung mit einem pH-Wert zwischen pH 3 und pH 7 bilden.

8. Wundversorgungsprodukt zur Verwendung nach Anspruch 7, umfassend
(a) eine Wundauflage
(b) optional eine Wundkontaktschicht
(c) optional eine Deckschicht, und
(d) optional eine Klebeschicht.

9. Wundversorgungsprodukt zur Verwendung nach Anspruch 7 oder 8, wobei die Puffersubstanzen in einer Menge von 1 bis 100 mmol pro cm² des Wundversorgungsprodukts vorliegen.

10. Wundversorgungsprodukt zur Verwendung nach einem der Ansprüche 7 bis 9, wobei die Wundauflage (a) einen Schaumstoff, ein Gittertüll, ein Gewebe, ein Gewirk und/oder ein Vlies enthält.

11. Wundversorgungsprodukt zur Verwendung nach einem der Ansprüche 7 bis 10, wobei das Wundversorgungsprodukt ein Hydrogel als Wundkontaktschicht (b) umfasst.

12. Wundversorgungsprodukt zur Verwendung nach einem der Ansprüche 7 bis 11, wobei die Puffersubstanzen in fester Form vorliegen und bevorzugt in der Wundauflage und/oder der Wundkontaktschicht verteilt sind.

## Claims

1. Buffer substances for use in the treatment of wounds, wherein the treatment being a phase-specific wound treatment is exclusively carried out during the granulation phase and comprises suppression of fibrin generation and/or fibrin cross-linking, and wherein the buffer substances, after dissolution in demineralised water at 37°C, form a buffer solution having a pH between pH 3 and pH 7.

2. Buffer substances for use according to claim 1, wherein the treatment comprises increasing the oxygen supply of the wound.

3. Buffer substances for use according any one of the preceding claims, wherein the treatment comprises inhibiting thrombin.

4. Buffer substances for use according to any one of the preceding claims, wherein the wound is a wound undergoing secondary healing, preferably a mechanical wound, a thermal wound, a wound caused by chemical substances or by irradiation.

5. Buffer substances for use according to any one of the preceding clams, wherein the buffer substances consist of an organic acid having 2 to 12 carbon atoms and the salt, preferably the alkali salt, of this acid.

6. Buffer substances for use according to any one of the preceding clams, wherein the buffer substances are selected from benzoic acid/benzoate, lactic acid/lactate, glyceric acid/glycerate, gluconic acid/gluconate, acetic acid/acetate, citric acid/citrate, aconitic acid/aconitate, glutaric acid/glutarate, tartaric acid/tartrate, malic acid/malate, succinic acid/succinate and glutamic acid/glutamate, and mixtures thereof.

7. Wound treatment product containing buffer substances for use in the treatment of wounds undergoing secondary healing exclusively during the granulation phase for the suppression of fibrin generation and/or fibrin crosslinking, wherein the buffer substances, after dissolution in demineralised water at 37°C, form a buffer solution having a pH between pH 3 and pH 7.

8. Wound treatment product for use according to claim 7, comprising
(a) a wound dressing
(b) optionally a wound contact layer
(c) optionally a cover layer, and
(d) optionally an adhesive layer.

9. Wound treatment product for use according to any one of claims 7 or 8, wherein the buffer substances are present in an amount of 1 to 100 mmol per cm² of the wound treatment product.

10. Wound treatment product for use according to any one of claims 7 to 9, wherein the wound dressing (a) contains a foam, lattice tulle, a woven fabric, a knitted fabric and/or a nonwoven.

11. Wound treatment product for use according to any one of claims 7 to 10, wherein the wound treatment product comprises a hydrogel as the wound contact layer (b).

12. Wound treatment product for use according to any one of claims 7 to 11, wherein the buffer substances are present in solid form and are preferably distributed in the wound dressing and/or wound contact layer.

## Revendications

1. Substances tampon pour utilisation dans le traitement de plaies, ledit traitement, en tant que traitement dans la phase correcte de la plaie, n'ayant lieu que dans la phase de granulation et comprenant la suppression de formation de fibrine et/ou de réticulation de fibrine, lesdites substances tampon formant une solution tampon ayant un pH de pH 3 à pH 7 après dissolution dans de l'eau déminéralisée à 37°C.

2. Substances tampon pour utilisation selon la revendication 1, le traitement comprenant une augmentation de l'apport d'oxygène dans la plaie.

3. Substances tampon pour utilisation selon l'une quelconque des revendications précédentes, le traitement comprenant l'inhibition de thrombine.

4. Substances tampon pour utilisation selon l'une quelconque des revendications précédentes, la plaie étant une plaie du deuxième degré en voie de guérison, de préférence une plaie mécanique, une plaie thermique, une plaie provoquée par des substances chimiques on par du rayonnement.

5. Substances tampon pour utilisation selon l'une quelconque des revendications précédentes, les substances tampon étant composées d'un acide organique de 2 à 12 atomes carboniques et d'un sel, de préférence le sel alcalin dudit acide.

6. Substances tampon pour utilisation selon l'une quelconque des revendications précédentes, les substances tampon étant choisies parmi l'acide benzoïque/benzoate, l'acide lactique/lactate, l'acide glycérique/glycérate, l'acide gluconique/gluconate, l'acide acétique/acétate, l'acide citrique/citrate, l'acide aconitique/aconitate, l'acide glutarique/glutarate, l'acide tartarique /tartrate, l'acide malique/malate, l'acide succinique/succinate et l'acide glutamique/glutamate, et leurs mélanges.

7. Produit de traitement des plaies contenant des substances tampon pour utilisation dans le traitement de plaies du deuxième degré en voie de guérison exclusivement dans la phase de granulation pour supprimer la formation de fibrine et/ou la réticulation de fibrine, lesdites substances tampon formant une solution tampon ayant un pH de pH 3 à pH 7 après dissolution dans de l'eau déminéralisée à 37°C.

8. Produit de traitement des plaies selon la revendication 7, comprenant
(a) une couverture pour plaie
(b) en option une couche en contact avec la plaie
(c) en option une couche supérieure, et
(d) une couche adhésive.

9. Produit de traitement des plaies selon l'une quelconque des revendications 7 ou 8, les substances tampon étant présentes dans une quantité de 1 à 100 mmol par cm² du produit de traitement des plaies.

10. Produit de traitement des plaies selon l'une quelconque des revendications 7 à 9, la couverture pour plaie (a) contenant une mousse de plastique, une tulle grillage, un tissu, un tricot et/ou un non-tissé.

11. Produit de traitement des plaies selon l'une quelconque des revendications 7 à 10, le produit de traitement des plaies comprenant un hydrogel en tant que couche en contact avec la plaie (b).

12. Produit de traitement des plaies selon l'une quelconque des revendications 7 à 11, les substances tampon étant présentes sous forme solide et, de préférence, distribuées dans la couverture pour plaie et/ou la couche en contact avec la plaie.
